# EUROPEAN PATENT APPLICATION

(11) **EP 4 797 287 A1**
(43) Date of publication of application: **26.08.2026**
(21) Application number: 26157969.2
(22) Date of filing: 11.02.2026
(51) Int. Cl.: G21F 3/02

(54) **DEVICE FOR PERSONAL PROTECTION AGAINST IONIZING RADIATION, HIGHLY BREATHABLE AND QUICK-DRYING**

(30) Priority: 24.02.2025 IT 202500003588
(71) Applicant: Simad S.r.l., 40066 Pieve di Cento (BO) (IT)
(72) Inventor: FALLAVENA, Franco, 40066 Pieve di Cento (BO) (IT)
(74) Representative: Gregorj S.r.l.

(57) **Abstract**

The present invention refers to a device for personal protection against ionizing radiation (1), in particular against X-rays, wearable by a user and comprising:
- an outer layer (2) forming an outer surface of the device for personal protection against ionizing radiation,
- an inner layer (3) forming an inner surface opposed to the outer surface, under conditions of use of the device the inner surface being intended to face, and/or come into contact with, the user,
- a protective core (23), in particular a shielding foil, suitable for shielding against ionizing radiation, the protective core being arranged between the outer layer and the inner layer.

The device (1) also comprises a drainage insert (4) configured to drain water and/or other liquids which may possibly have accumulated between the outer layer and the inner layer. The drainage insert (4) is cross-linked.

## Description

### Technical field of the invention

The present invention refers to a device for personal protection against ionizing radiation, particularly against X-rays, having characteristics such as being highly breathable and quick-drying.

The wording "device for personal protection" means equipment destined to be worn by an individual to protect him/her from one or more risks susceptible to impair the safety or health, and also any complement or accessory destined to this purpose. Particularly, the present invention refers to personal protective devices against ionizing radiation, particularly X-rays.

### Prior art

Devices for personal protection against ionizing radiation are known. Ionizing radiation is a type of radiation which has sufficient energy to directly or indirectly ionize the atoms or the molecules which it interacts with, so as to determine alterations of the electron structure of them. Such radiation is particularly dangerous if an individual is subjected to a repeated prolonged exposure. In fact, it can affect biological tissues, determining biological damages which can be also serious, particularly tumors. Examples of ionizing radiation are α-rays, β-rays, γ-rays, and X-rays.

Referring particularly to X-rays, they are exploited, as it is known, in healthcare environments (such as hospitals, medical offices, clinics, dentist's offices) for diagnostic purposes, for example for taking radiographies. Moreover, artificial radioactive sources are used for a therapeutical purpose.

Since the ionizing radiation is potentially dangerous, it is essential that, when radiographies are taken, the X-rays hit only those portions of the body of an individual on which a radiologic probe must be performed. Therefore, the zones of the body of the patient which are not involved in the radiography must be protected.

A further particularly perceived requirement is one that consists of protecting the operators, such as the radiology operators, technicians, doctors from the X-ray exposure. In fact, they, due to their professions, are repeatedly exposed to radiation, therefore it is essential to provide systems adapted to protect them.

In order to protect patients and operators, it is known the use of suitable personal protection devices. They can, for example, comprise aprons, skirts, vests, full aprons, collars, thyroid protection elements, shrugs, hand covers, aprons for intraoral exams, as a function of the part of the body to be protected. Such personal protection devices comprise a core made of a radiation shielding material. For example, they can be made of lead, which is particularly adapted for the protection against X-rays, or they are made of lead rubber or materials alternative to lead for manufacturing devices for personal protection against ionizing radiation. The core of the shielding material is wrapped by an enclosure, for example made of fabric, the core being housed inside the enclosure.

Personal protection devices, particularly aprons or similar, are generally heavy and adherent to the body, consequently the user is subjected to high perspiration.

Moreover, since the personal protection devices are for example used in operating rooms and analogous environments, they can be soiled by blood and other organic liquids because they often come in contact with them.

Therefore, a particularly felt need consists of washing and suitably sanitizing the personal protection devices. However, the personal protection devices, due to their conformation, are difficult to be correctly washed. In fact, when the washing is made by immersion or is performed by falling water, water enters the space inside the enclosure, where the core is housed so that water cannot quickly outflow from the enclosure seams. Therefore, the step of drying the personal protection device can require a few days, during this period the personal protection device cannot be used.

Alternatively, the personal protection devices are simply washed by means of sponges soaked in water and/or detergent. However, this type of washing is not capable of ensuring the sanitization of the personal protection device because it is superficial and consequently approximate.

In order to overcome the beforehand cited problems, the Italian patent 1417194 in the name of the same Applicant is known, which discloses personal protection devices against ionizing radiation which comprise an enclosure whose interior houses the ionizing radiation protective core and means for draining water and other liquids. The drainage means are provided with openings formed in the enclosure of the personal protection device and they are arranged at the lower portions of the personal protection device (in case of an apron, they are located at portions facing the user's legs under conditions of use).

The Applicant has observed that this technical solution could be further improved.

### Objects of the invention

Therefore, the main object of the present invention consists of overcoming the drawbacks beforehand described with reference to the prior art.

Moreover, an object of the present invention consists of providing a personal protection device against ionizing radiation having quick drying features.

A further object of the invention consists of providing a personal protection device against ionizing radiation being simultaneously characterized by quick drying and by high transpiration capacity.

Moreover, the invention provides a highly effective technical solution alternative to the ones of the prior art.

These and other objects are met by a personal protection device against ionizing radiation in accordance with the following description and the attached claims.

### Conventions and definitions

It is noted that in the following detailed description corresponding parts/components/elements are indicated by the same numeral references. The figures could illustrate the object of the invention by not-to-scale representations; therefore, parts/components/elements illustrated in the attached figures and regarding the object of the invention, could only refer to schematic representations. Using "/" in the present text means "and/or", if it is not clearly indicated otherwise or unless otherwise specified.

In the context of the present disclosure, the use of terms such as "on", "top", "upper", "below", "bottom", "lower", "alongside", "lateral", "laterally", "inner", "internally", "outer", "externally", "horizontal", "horizontally", "vertical", "vertically", "frontal", "frontally", "posterior", "posteriorly", "right", "left", similar terms and corresponding variants refer, except for specific different indications, to at least one spatial orientation that the object of the invention can take under conditions of use; the terms refer to what is shown in the drawings, illustrating the orientation associated to a normal use of the invention. Such terms are used only for helping the reader and are not limiting.

### Brief description of the drawings

In order to better comprehend the invention and appreciate the advantages, some embodiments thereof will be described in an exemplifying and not limiting way in the following with reference to the attached figures.

A brief description of the attached figures follows:
Figure 1 illustrates a view of a personal protection device (an apron) against ionizing radiation in accordance with a possible embodiment of the invention, and on the right a detail showing the arrangement of the drainage insert between the inner layer and the outer layer;
Figure 2 closely illustrates the drainage insert of the personal protection device against ionizing radiation of Figure 1.

### Detailed description of embodiments of the invention

A device for personal protection against ionizing radiation, particularly against X-rays, in accordance with the invention, is generally indicated in the Figures by the numeral reference 1. Examples of ionizing radiations against which the device 1 is capable of protecting a user are α-rays, β-rays, γ-rays, and X-rays.

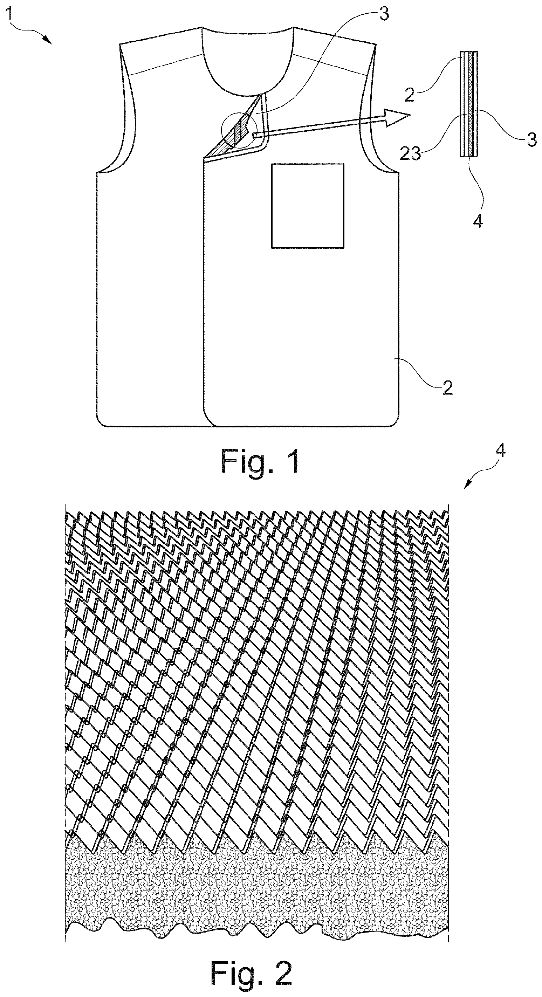

In the present description, reference will be mainly made to a personal protection device to be used in the healthcare field, adapted to protect the user against X-rays. Still more particularly, reference will be made to an apron (see Figure 1) which can be worn, for example, by a healthcare operator usually working in proximity to X-ray emitters or by a patient. However, it is noted that the sanitary apron for protection against X-rays must be understood in a non-limiting way as a simple example of a personal protection device against ionizing radiation. For example, the device 1 can be a skirt, a vest, a full apron, a glove, an arm cover, a thyroid protection element, a shrug, a hand cover, an apron for intraoral exams, or similar.

In the following, reference will be made to the personal protection device against ionizing radiation, particularly against X-rays, simply as "PPE" or "device".

The device 1 is wearable by a user and comprises:
- an outer layer 2 forming an outer surface of the device,
- an inner layer 3 forming an inner surface opposed to the outer surface, the inner surface being destined to face an/or come in contact with the user under conditions of use of the device 1,
- a protective core 23 adapted to shield against ionizing radiation and arranged between the inner layer 2 and the outer layer 3.

Preferably, the outer layer 2 is waterproof.

The purpose of the protective core is to shield away the ionizing radiation and is configured to mitigate such radiation in order to prevent it from being transmitted to the user when this latter wears the device 1. With reference to the apron, the protective core can be, for example, shaped as a shielding sheet or foil apt to make the apron wearable and is preferably made by a material suitable for shielding against X-rays. Materials suitable for shielding against X-rays or other ionizing radiation are known per se. Examples of possible materials for shielding against X-rays are lead rubber or bilayer rubbers made of antimony and bismuth; obviously other material adapted to the purpose can be provided.

By their nature, the outer layer 2 and the inner layer 3 are not adapted per se to shield against ionizing radiation and are preferably made of fabric, for example waterproof polyamide. The device 1 can be provided with further components for improving the wearability and usability thereof, such as: paddings, zippers, buttons, closure drawstrings, sleeves, one or more pockets, belts, or similar.

Further, the device 1 comprises a drainage insert 4 configured to drain water or other liquids possibly accumulating between the outer layer 2 and the inner layer 3. The drainage insert 4 is arranged between the inner layer 3 and the outer layer 2, and has a mesh-like conformation in order to promote the drainage or outflow of a liquid accumulating between the outer layer and the inner layer, from the PPE. As illustrated in Figure 2, the drainage insert 4 can have an undulated conformation and/or can define a sort of zig-zag pattern on the surface; substantially, the drainage insert 4 provides plural overlapped reticulations supported by a suitable mesh. Preferably, the drainage insert 4 provides both substantially horizontally arranged reticulations and substantially vertically arranged reticulations. The above mentioned undulated conformation provides the structure with a greater area by which it is possible to simultaneously have perspiration and greater flexibility, the latter being fundamental in order to provide the PPE 1 with good ergonomics.

The drainage insert 4 is arranged between the inner layer 3 and the outer layer 2, therefore it does not define drainage openings formed in the inner layer 3 and in the outer layer 2. In other words, the drainage insert 4 is not visible from the outside of the device 1.

The drainage insert 4 has a mesh-like conformation: in other words, the drainage insert 4 provides openings defining a mesh-like structure, i.e. defining a mesh. The Applicant has verified that the arrangement of the mesh-like drainage insert 4 (drainage mesh), as hereinbefore specified and particularly between the inner layer 3 and protective core, has improved the drying time of the PPE, substantially reducing the time required to dry it.

Preferably, the drainage insert is arranged between the inner layer 3 and the protective core. By arranging the drainage insert 4 between the inner layer 3 and the protective core the invention enables to separate the fabric inner layer from the shielding foil, thus reducing even more the drying time. The drying time is reduced because the gap between the fabric and the shielding foil promotes evaporation. It is observed that the choice of internally arranging the drainage insert 4 is not only connected to the reduction of the drying time, but also by the possibility of increasing the perspiration of the PPE 1 in accordance with the invention, ensuring in this way an increase of the comfort for a user wearing the PPE 1.

Preferably, the drainage insert 4 is cross-linked.

More particularly, the drainage insert 4 can comprise a cross-linked fabric (see Figure 2). Preferably, the cross-linked fabric is made of plastic and/or polymeric material. Polyethylene is a material suitable for the drainage insert.

In accordance with a preferred embodiment, the drainage insert 4 is in the form of a fabric layer, that is preferably cross-linked, arranged between the inner layer 3 and the protective core.

The drainage insert 4 is arranged substantially without interruption between the inner layer 3 and the outer layer 2, as hereinbefore said preferably between the inner layer 3 and the protective core, possibly except for some specific areas, for example an area of one or more shoulder pads. Substantially, some areas of the PPE can be devoid of the drainage insert 4.

Since the PPE has a layered structure (see Figure 1 and the corresponding detail), the drainage insert develops in accordance with the development of the inner surface and/or of the outer surface, particularly of both, and follows their development or conformation. In specific terms, since the inner layer 3, the outer layer 2, and the drainage insert 4 are made of light and deformable materials (note that the PPE is wearable), these three layers can be oriented in the same way, therefore the drainage insert 4, which is an intermediate layer, follows the development and deformations, folds, etc. of the inner layer 3 and of the outer layer 2.

Consequently, the drainage insert 4 develops for a prevailing part of the development of the device 1, and preferably for the entire development of the device 1, possibly except for some specific areas, for example an area of one or more shoulder pads.

The drainage insert 4 is thin and light, its presence improves the wearability of the PPE 1, also due to an improved perspiration, as hereinbefore described. Further details follow.

Preferably, the drainage insert 4 is in the shape of a layer having a thickness less than 1 cm, preferably less than 50 mm or less than 5 mm.

In a preferred embodiment, the drainage insert 4 is in the shape of a layer having a thickness comprised between 1 mm and 5 mm, preferably comprised between 2 and 4 mm. In accordance with such reduced thickness, the PPE 1 is compact and shows a limited size in terms of its total thickness, in other words of the thickness of the layered structure provided with, from the inside to the outside: inner layer, drainage insert, shielding foil, and outer layer.

In some embodiments, the drainage insert 4 can be arranged between the inner layer 3 and the protective core and, in addition or as an alternative, between the outer layer 2 and the protective core. Preferably, the drainage insert 4 is exclusively arranged between the inner layer 3 and the protective core.

In the specific embodiment illustrated in the attached Figures, the drainage insert 4 is shaped as a mesh and is made of a cross-linked material (for example polyester). Moreover, it is arranged between the shielding foil and the inner layer 3 of the device and it develops without interruption between them, substantially for the entire development of the device, except for the area of the shoulder pads. It is observed that the area of the shoulder pads can be devoid of drainage inserts 4 because in that area the PPE 1 is already provided with a waterproof sponge, increasing the comfort of the PPE 1. Further, it is observed that the PPEs 1 are clothes which are typically vertically arranged during the drying step, consequently water drops by gravity and accumulates into the lower edge, at which a further drainage element can be provided, which will be described in the following and which enables the outflow of water or other liquid.

In addition, the device 1 can comprise at least one further drainage element. Preferably, the further drainage element is arranged, particularly exclusively arranged, at portions or edges or edge portions of the device 1, for example at lower portions or edges of the device; in case of an apron the lower portions are portions facing the legs of the user under conditions of use. Such further drainage element can have openings defined in the outer layer 2 of the PPE and therefore it can be substantially analogous to the drainage means of the Italian Patent 1417194 in the name of the same Applicant. Figure 1 does not show the further drainage element. Preferably, the further drainage element is in the shape of a mesh and is configured to drain water or other liquid possibly accumulated inside the PPE 1, particularly between the outer layer and the shielding foil and consequently enables to quickly dry the PPE after the washing step.

The presence of the further mesh-shaped drainage element and the provision of the drainage insert therefore enable to separate the fabric inner layer from the shielding foil, further reducing the drying times.

### Further advantages and final notes

The provision of the drainage insert 4 enables high perspiration because the gap, formed between the adjacent layers, thanks to it (perforated/cross-linked fabric) enables the moisture to move away and, thanks to the movements of the PPE's user and therefore of the PPE, the gap aids in removing the moisture outside the PPE, thus contributing in this way to reduce the generated body heat.

The drainage insert 4 provided by the device 1 in accordance with the invention is waterproof and soft, for example is made of polyester.

Such device 1 in accordance with the invention has a dual function: it fulfills the regulatory requirements of the regulation UE 2016/425 regarding the ergonomics of the PPE and facilitates the drying of the fabric inner layer, which is the most difficult to treat/dry.

Since the drainage insert 4 has a mesh-like structure, it is provided with a perforated weft capable of enabling water, which during the washing step by immersion or water fall inevitably penetrates inside the gap present between the protective core and the covering fabric of the PPE (therefore between the outer layer 2 and the inner layer 3) to easily outflow.

The protection given by the claims extends to each element, component and/or step of the invention equivalent to the one/s that is/are claimed.

It is understood that each element, component and/or step of the product/method according to the invention can be substituted with an equivalent element, component and/or step (in the following, "equivalent"); such equivalent/s can be already existent at the filing or priority date of the present patent text or can be of subsequent conception/development.

## Claims

1. Device (1) for personal protection against ionizing radiation, in particular from X-rays, configured to be worn by a user and comprising:
- an outer layer (2) forming an outer surface of the device for personal protection against ionizing radiation,
- an inner layer (3) forming an inner surface opposed to the outer surface, under conditions of use of the device the inner surface being intended to face, and/or come into contact with, the user,
- a protective core (23), in particular a shielding foil, suitable for shielding against ionizing radiation, the protective core (23) being arranged between the outer layer (2) and the inner layer (3),
in which the device (1) also includes a drainage insert (4) configured to drain water and/or other liquid that may have accumulated between the outer layer (2) and the inner layer (3),
and in which the drainage insert (4) has a mesh-like conformation and is arranged between the inner layer (3) and the outer layer (2).

2. Device according to claim 1, wherein the drainage insert (4) comprises a cross-linked fabric, for example of plastic and/or polymeric material, in particular polyethylene.

3. Device according to claim 1 or 2, wherein the drainage insert (4) is in the form of a fabric layer.

4. Device according to claim 3, wherein the drainage insert (4) in the form of a fabric layer is arranged between the inner layer and the protective core.

5. Device according to claim 1 or 2 or 3 or 4, wherein the drainage insert (4) is arranged substantially without interruption between the inner layer (3) and the outer layer (2),
possibly except for some specific areas, e.g. an area of one or more shoulder pads.

6. Device according to claim 1 or 2 or 3 or 4 or 5, wherein the drainage insert (4) is arranged substantially without interruption between the inner layer (3) and the protective core (23),
possibly except for some specific areas, e.g. an area of one or more shoulder pads.

7. Device according to claim 1 or 2 or 3 or 4 or 5 or 6, wherein the drainage insert (4) develops in accordance with the development of the inner surface and/or the outer surface, preferably the drainage insert (4) following the development of the inner surface and/or of the outer surface.

8. Device according to any one of the preceding claims, wherein the drainage insert (4) develops for a prevailing part of a development of the device (1), preferably the drainage insert (4) developing substantially for the entire development of the device (1),
possibly except for some specific areas, e.g. an area of one or more shoulder pads.

9. Device according to any one of the preceding claims, wherein the drainage insert (4) is in the form of a layer having a thickness of less than 1 cm, preferably less than 5 mm.

10. Device according to any one of the preceding claims, wherein the drainage insert (4) is in the form of a layer having a thickness between 1 mm and 5 mm, preferably between 2 mm and 4 mm.

11. Device according to any one of the preceding claims, wherein the drainage insert (4) is arranged between the inner layer (3) and the protection core (23).

12. Device according to any one of the preceding claims, wherein the drainage insert (4) is arranged between the outer layer (2) and the protection core (23).

13. Device according to any one of the preceding claims, the drainage insert (4) being arranged exclusively between the inner layer (3) and the protective core (23).

14. Device according to any one of the preceding claims, further comprising at least one further drainage element.

15. Device according to claim 14, said at least one further drainage element being arranged, in particular exclusively arranged, at portions or edges or edge portions of the device (1),
in particular the further drainage element providing openings that are defined in the outer layer (2).
